# EUROPEAN PATENT APPLICATION

(11) **EP 4 108 186 A1**
(43) Date of publication of application: **28.12.2022**
(21) Application number: 22181097.1
(22) Date of filing: 24.06.2022
(51) Int. Cl.: A61B 17/00, A61B 17/29, A61B 18/12, A61B 17/115, A61B 18/00

(54) **UNIVERSAL MOTOR CARTRIDGE FOR REPOSABLE SURGICAL INSTRUMENT**

(30) Priority: 25.06.2021 US 202163214985 P; 10.06.2022 US 202217837152
(71) Applicant: Covidien LP, Mansfield, MA 02048 (US)
(72) Inventor: BONN, Kenlyn S, Boulder, 80301 (US); BAGROSKY, Tyler J., Boulder, 80301 (US)
(74) Representative: Maschio, Antonio

(57) **Abstract**

A universal motor cartridge for a surgical instrument includes a reusable housing having a distal face and a bottom surface and configured to securely engage a disposable surgical instrument. Couplers disposed on the distal face are adapted to engage couplers disposed on the surgical instrument upon engagement of the housing. A plurality of electrical interfaces on the bottom surface are adapted to electrically engage corresponding electrical interfaces on the surgical instrument, such that, upon activation of each switch disposed on the surgical instrument, the corresponding electrical interface on the surgical instrument communicates with the corresponding electrical interface on the bottom surface of the housing, which, in turn, actuates the corresponding coupler to engage and actuate the respective coupler on the surgical instrument causing the surgical instrument to rotate/articulate a shaft, open/close a pair of jaw members, deform staples, move a knife or energize an end effector.

## Description

### CROSS REFERENCE TO RELATED APPLICATION

This application claims the benefit of U.S. Provisional Application Serial No. 63/214,985 filed June 25, 2021, the entire contents of which being incorporated by reference herein.

### BACKGROUND

### 1. Technical Field

The present disclosure relates generally to the field of surgical instruments. In particular, the disclosure relates to a universal motor cartridge for use with multiple endoscopic electrosurgical instruments.

### 2. Background of Related Art

Various electrosurgical instruments are commonly used in open and endoscopic surgical procedures to coagulate, cauterize, seal, staple or otherwise treat tissue. Each of these instruments utilize a combination of handles, triggers, rotating assemblies, firing assemblies and energy activation buttons or levers to accomplish this purpose and, as such, many of these instruments are customized with various types of mechanical mechanisms for actuating the various components associated therewith depending upon the particular surgical requirement, e.g., sealing tissue versus stapling tissue.

Advancements in instrumentation and electronics have led manufacturers to develop instrumentation that rely on electrical actuators and components to off-load the forces required to accomplish certain repetitive and high force tasks, e.g., stapling, compressing tissue, etc. These instruments are especially advantageous during long surgical procedures to reduce surgical fatigue.

In some instances it may be desirable to manufacture a reposable universal motor cartridge that is configured to engage the electrical actuators of a variety of different types of endoscopic surgical instruments.

### SUMMARY

The present disclosure relates generally to the field of surgical instruments. In particular, the disclosure relates to an endoscopic electrosurgical forceps that includes a system and method for springing open jaw members.

As is traditional, the term "distal" refers herein to an end of the apparatus that is farther from an operator, and the term "proximal" refers herein to the end of the electrosurgical forceps that is closer to the operator.

The present disclosure relates to a universal motor cartridge for a surgical instrument and includes a reusable housing having a distal face at a distal end thereof and a bottom surface, the housing configured to securely engage a disposable surgical instrument. A plurality of couplers is disposed on the distal face, one or more of the plurality of couplers adapted to engage a corresponding coupler disposed on the surgical instrument upon engagement of the housing thereto.

A plurality of electrical interfaces is disposed on the bottom surface, one or more of the plurality of electrical interfaces is adapted to electrically engage a corresponding electrical interface disposed on a mating surface of the surgical instrument. Upon activation of one of the switches disposed on the surgical instrument, the corresponding electrical interface on the surgical instrument communicates with the one of the plurality of electrical interfaces on the bottom surface of the housing, which, in turn, actuates the one of the plurality of couplers to engage and actuate one corresponding coupler on the surgical instrument. The corresponding coupler on the surgical instrument, upon actuation thereof, causes the surgical instrument to: rotate or articulate a shaft associated therewith, open or close a pair of jaw members associated therewith, deform a plurality of staples associated therewith, move a knife associated therewith to cut tissue, and/or energize an end effector associated therewith.

In aspects according to the present disclosure, the housing is configured to securely engage an electrosurgical forceps having a plurality of switches associated therewith, each switch of the plurality of switches, upon activation thereof, communicating with a corresponding one of the plurality of electrical interfaces on the electrosurgical forceps which, in turn, communicates with a corresponding electrical interface on the bottom surface of the housing to actuate a corresponding coupler on the housing which, in turn, actuates a corresponding coupler on the electrosurgical forceps to cause the electrosurgical forceps to: rotate or articulate a shaft associated therewith, open or close a pair of jaw members associated therewith, move a knife associated therewith to cut tissue, and/or energize an end effector associated therewith.

In aspects according to the present disclosure, the housing is configured to securely engage a stapling device having a plurality of switches associated therewith, each switch of the plurality of switches, upon activation thereof, communicating with a corresponding one of the plurality of electrical interfaces on the stapling device which, in turn, communicates with a corresponding electrical interface on the bottom surface of the housing to actuate a corresponding coupler on the housing which, in turn, actuates a corresponding coupler on the stapling device to cause the stapling device to: rotate or articulate a shaft associated therewith, open or close a pair of jaw members associated therewith, move a knife associated therewith to cut tissue, or deform a plurality of staples associated therewith.

In aspects according to the present disclosure, the motor cartridge is adapted to connect to an electrical energy source. In other aspects according to the present disclosure, the motor cartridge is adapted to connect to an internal electrical energy source.

In aspects according to the present disclosure, the housing includes one or more mechanical interfaces configured to align and securely engage a corresponding number of mechanical interfaces disposed on the surgical instrument.

In aspects according to the present disclosure, the housing includes one switch disposed thereon that actuates a corresponding coupler on the housing which, in turn, actuates a corresponding coupler on the surgical instrument to cause the surgical instrument to: rotate or articulate a shaft associated therewith, open or close a pair of jaw members associated therewith, deform a plurality of staples associated therewith, move a knife associated therewith to cut tissue, and/or energize an end effector associated therewith.

In aspects according to the present disclosure, the motor assembly is configured to identify the type of surgical instrument upon engagement therewith and configure one or more of the plurality of couplers disposed on the distal face in accordance thereto.

In aspects according to the present disclosure, the surgical instrument includes two or more switches disposed thereon, activation of a first of the two switches actuates a first coupler on the housing which, in turn, actuates a first coupler on the surgical instrument to cause the surgical instrument to perform a first function and activation of a second of the two or more switches actuates a second coupler on the housing which, in turn, actuates a second coupler on the surgical instrument to cause the surgical instrument to perform a second function and activation of a both the first and second switches of the two or more switches actuates a third coupler on the housing which, in turn, actuates a third coupler on the surgical instrument to cause the surgical instrument to perform a third function. In other aspects according to the present disclosure, the first, second and third functions of the surgical instrument include: rotation or articulation of a shaft associated with the surgical instrument, opening or closing a pair of jaw members associated with the surgical instrument, deforming a plurality of staples associated with the surgical instrument, moving a knife associated with the surgical instrument to cut tissue, or energizing an end effector associated with the surgical instrument. In still other aspects according to the present disclosure, simultaneous actuation of the first and second switches on the surgical instrument actuates the third coupler on the housing which, in turn, actuates the third coupler on the surgical instrument to cause the surgical instrument to perform the third function.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure.
FIG. 1A is a perspective view of an electrosurgical forceps adapted to couple to a universal motor cartridge according to the present disclosure;
FIG. 1B is an enlarged, perspective view of an end effector assembly of the embodiment shown in FIG. 1A
FIG. 2 is a perspective view of a surgical stapler adapted to couple to the universal motor cartridge according to the present disclosure; and
FIG. 3 is a perspective view of another electrosurgical forceps adapted to couple to the universal motor cartridge according to the present disclosure

### DETAILED DESCRIPTION

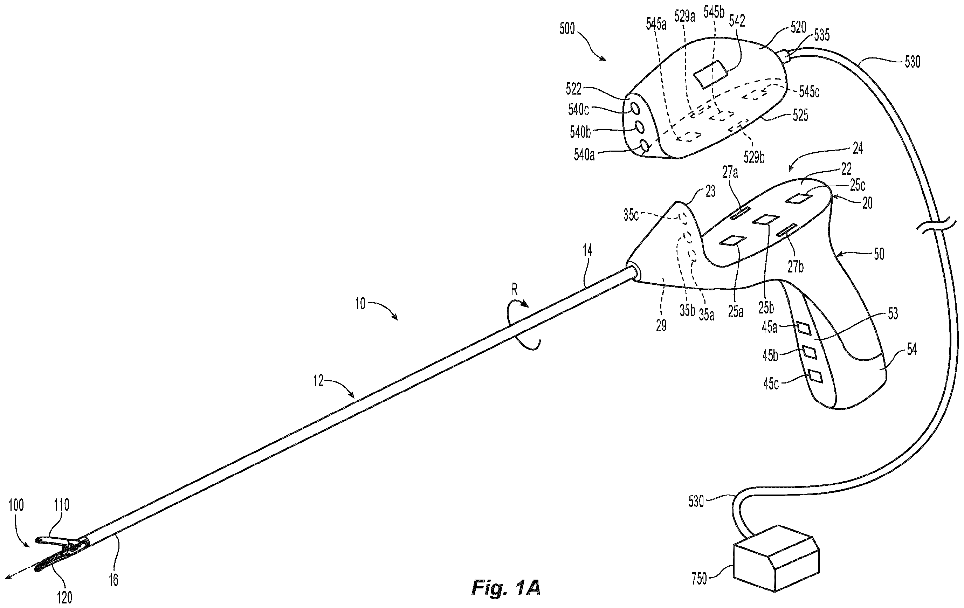

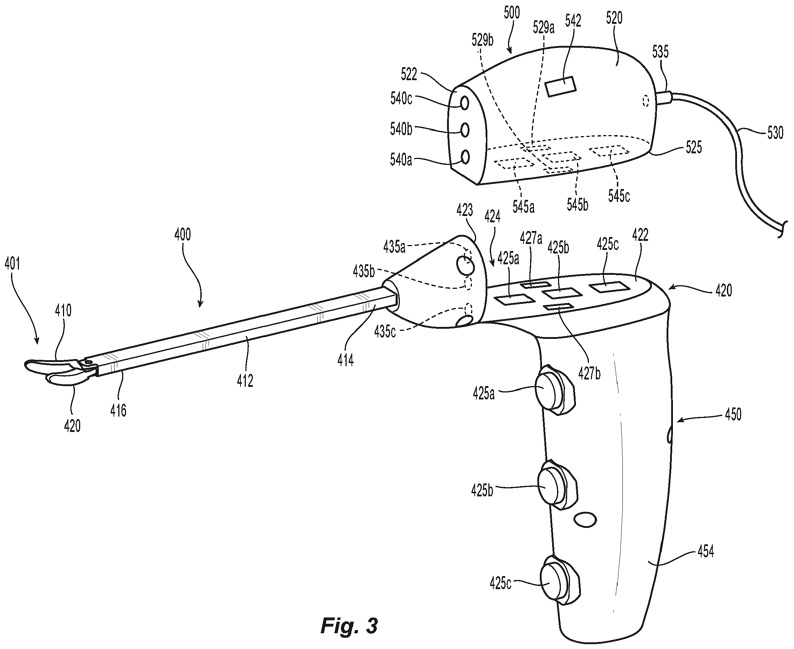

Turning initially to FIG. 1A, one embodiment of an endoscopic bipolar forceps 10 is shown for use with various surgical procedures and generally includes a housing 20, a handle 50, and an end effector assembly 100 which mutually cooperate to grasp, seal and divide tubular vessels and vascular tissue. For the purposes herein, forceps 10 is characterized as a bipolar forceps but may be utilized to seal tissue similar to forceps 400 described below with respect to FIG. 3. Moreover, forceps 10 is described in terms of an endoscopic instrument, however, it is contemplated that an open version of the forceps may also include the same or similar operating components and features as described below.

Forceps 10 includes a shaft 12 having a distal end 16 dimensioned to mechanically engage the end effector assembly 100 and a proximal end 14 that mechanically engages the housing 20. The proximal end 14 of shaft 12 is received within the housing 20. In the drawings and in the description which follows, the term "proximal", as is traditional, will refer to the end of the forceps 10 which is closer to the user, while the term "distal" will refer to the end which is further from the user.

Handle 50 depends from housing 20 and is configured to support a variety of actuation buttons, levers or switches 45a-45c on distal face 53 of handle 50, the purposes of which are explain in detail below. Handle 50 is integrally associated with housing 20. End effector assembly 100 includes a first jaw member 110 movable relative to a second jaw member 120 upon actuation of one of the actuation buttons, e.g., actuation button 45a. A cavity 24 is defined between an upper surface 22 (which mates with the bottom surface 525 of the motor cartridge 500 upon engagement thereof) of the housing 20 and a distal face 23 extending therefrom. Cavity 24 is configured to receive a motor cartridge 500 therein. One or more mechanical interfaces or alignment features 27a, 27b are utilized to operably engage and align forceps 10 with corresponding interfaces 529a, 529b disposed on the motor cartridge 500 to align and secure the motor cartridge 500 within cavity 24. Mechanical interfaces 27a, 27b may include inter-cooperating components, slide rails, tongue and groove arrangements, magnets, etc.

Upper surface 22 supports a plurality of electrical or electromechanical interfaces 25a-25c thereon which are configured to align and operably engage a plurality of electrical or electromechanical interfaces 545a-545c (shown in phantom) disposed on a bottom surface 525 of the motor cartridge 500. In embodiments, with the motor cartridge 500 disposed in the reusable housing, a variety of gears, pins, shafts, etc. configured to engage, align, or drive the various components in the disposable handle may be included depending upon a particular purpose. Mechanical interfaces 27a, 27b and 529a, 529b help to align the electrical or electromechanical interfaces 25a-25c and 545a-545c. It is important to note that the number of electrical or electromechanical interfaces 25a-25c associated with upper surface 22 of housing 20 may be different than the number of electrical or electromechanical interfaces 545a-545c associated with motor cartridge 500. In other words, not all instruments that are adapted to couple to the universal motor cartridge 500 will be adapted to utilize all of the electrical or electromechanical interfaces 545a-545c for specific purposes.

Motor cartridge 500 also includes a plurality of universal couplers 540a-540c disposed on a distal surface 522 thereof that are configured to operably engage one or more couplers 35a-35c disposed on distal face 23 of forceps 10 upon seating the motor cartridge 500 within cavity 24. As mentioned above, a variety of gears, pins, shafts, etc. configured to engage, align, or drive the various components in the disposable handle may be included in the motor cartridge 500 depending upon a particular purpose. Universal couplers 540a-540c are actuatable upon actuation of one or more actuation buttons 45a-45c. Each actuation button, e.g., button 45a, couples to a corresponding universal coupler, e.g., coupler 540a, via an electrical or electromechanical connection through the engagement of corresponding electrical or electromechanical interfaces, e.g., interface 25a and 545a.

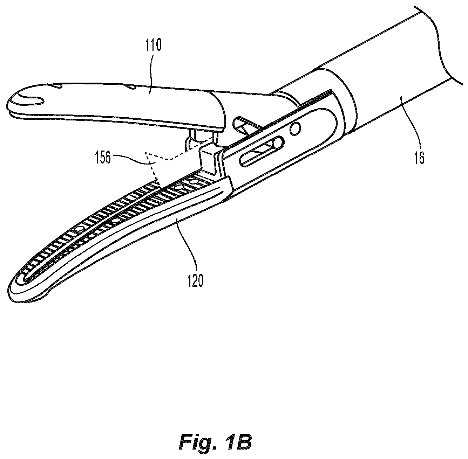

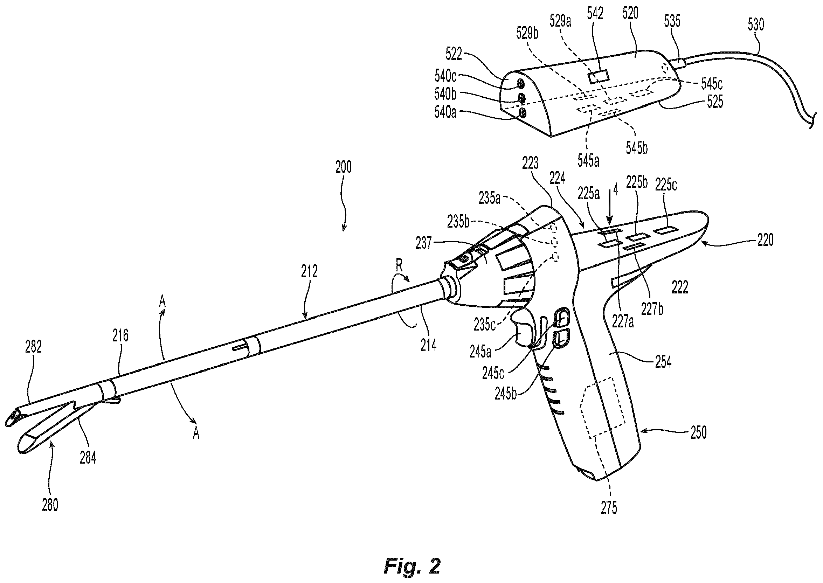

Any given instrument, e.g., forceps 10, may have any number of couplers to perform any number of functions, e.g., open and close the jaw members 110, 120, rotate the shaft/jaw members 110, 120, articulate the shaft 12, fire a staple into tissue (FIG. 2), actuate a knife 156 between jaw members (see FIG. 1B), and/or energize the jaw members 110, 120 to treat tissue. The universal nature of the motor cartridge 500 with the plurality of couplers 540a-540c enables instrument manufacturers the ability to utilize the plurality of couplers 540a-540c to perform any number of tasks for various surgical instrumentation. As such, the motor cartridge 500 may be configured to identify the type of surgical instrument, e.g., forceps 10, and configure the various couplers 540a-540c with respective switches 45a-45c in accordance therewith. Moreover, instruments may be manufactured such that two or more couplers, e.g., 540a, 540b, may be actuated at the same time (or sequentially) upon actuation of one or more actuation buttons 45a-45c (again at the same time or sequentially), enabling more versatility with the universal motor cartridge 500. As can be appreciated various control systems 29 and/or mechanical components, e.g., compound gearing systems (not shown), may be utilized to accomplish this purpose.

An electrical cable 530 extends from the motor cartridge 500 and is adapted to connect to an electrical energy source, e.g., generator 750. Cable 530 may be selectively engageable with motor cartridge 500 at connection 535. In embodiments, the cable 530 may be disconnected at connection 535 if the instrument, e.g., stapler 200 (FIG. 2), includes an internal power supply 275 (shown in phantom).

To mechanically control the jaw members 110, 120 of the end effector assembly 100, the housing 20 supports the various switches or buttons 45a-45c which are individually configured to perform different tasks: open and close the jaw members 110, 120, rotate or articulate the shaft 12, advance the knife 156 between jaw members 110, 120 (See FIG. 1B), energize the jaw members 110, 120 to treat tissue, etc. As mentioned above, the various switches 45a-45c electrically and/or mechanically connect ultimately to the corresponding couplers 35a-35c through the motor cartridge 500.

The various switches 45a-45c may be customized for each particular instrument, e.g., forceps 10, and, in instances, configured as per user preference upon startup. For example, switch 45a may be a toggle switch and may be configured to open and close the jaw members 110, 120 upon actuation thereof. Switch 45b may be used to rotate the shaft 12 in the direction "R" and switch 45c may be utilized to advance the knife 156 (See FIG. 1B). Another switch (not shown) may be disposed on the housing 20 or handle 50 and used to energize the jaw members 110, 120 to treat or seal tissue. Motor cartridge 500 may also include one or more switches 542 which may be customized for any purpose (rotation, articulation, opening/closing jaw members 110, 120, a safety switch, energizing jaw members 110, 120, etc.).

In use, the user unpacks a sterilized disposable instrument, e.g., forceps 10, and couples the reusable universal motor cartridge 500 to the forceps 10 as described above. Once coupled, the user may be prompted to configure the forceps 10 for use or use the default settings depending upon a particular purpose. A user's profile may be stored in the motor cartridge 500 for a given instrument. After use, the disposable portion, e.g., forceps 10, is discarded or stored for sterilization and refurbishment. The reusable motor cartridge 500, once sterilized, may be used again with a new forceps 10, vessel sealer 400 (FIG. 3) or stapler 200 (FIG. 2).

Turning now to FIG. 2, the universal motor cartridge 500 may be used with a disposable stapler 200 in much the same fashion as described above. Any stapler 200 may be configured for use with motor cartridge 500 such as, for example, the staplers described with reference to U.S. Patent No. 6,241,139 the entire contents of which are incorporated by reference herein. Stapler 200 includes a housing 220 having a shaft 212 extending therefrom which has a distal end 216 dimensioned to mechanically engage the stapler assembly 280 and a proximal end 214 which mechanically engages the housing 220. The proximal end 214 of shaft 212 is received within the housing 220. A handle 250 depends from housing 220 and is configured to support a variety of actuation buttons, levers or switches 245a-245c on handle 250, the purposes of which are explain in detail below. Handle 250 is integrally associated with housing 220. Stapler assembly 280 includes a first jaw member 282 movable relative to a second jaw member 284 upon actuation of one of the actuation buttons, e.g., actuation button 245a.

A cavity 224 is defined between an upper surface 222 of the housing 220 and a distal face 223 extending therefrom. Cavity 224 is configured to receive the motor cartridge 500 therein. One or more mechanical interfaces or alignment features 227a, 227b are utilized to operably engage and align 200 with corresponding interfaces 529a, 529b disposed on the motor cartridge 500 to align and secure the motor cartridge 500 within cavity 224. Mechanical interfaces 227a, 227b may include inter-cooperating components, slide rails, tongue and groove, magnets, etc.

Upper surface 222 supports a plurality of electrical or electromechanical interfaces 225a-225c thereon which are configured to align and operably engage a plurality of electrical or electromechanical interfaces 545a-545c (shown in phantom) disposed on the motor cartridge 500. Mechanical interfaces 227a, 227b and 529a, 529b help to align the electrical or electromechanical interfaces 225a-225c and 545a-545c. It is important to note that the number of electrical or electromechanical interfaces 225a-225c associated with upper surface 222 of housing 220 may be different than the number of electrical or electromechanical interfaces 545a-545c associated with motor cartridge 500. In other words, not all instruments that are adapted to couple to the universal motor cartridge 500 will be adapted to utilize all of the electrical or electromechanical interfaces 545a-545c for specific purposes.

Motor cartridge 500 also includes the plurality of universal couplers 540a-540c disposed on the distal surface 522 thereof that are configured to operably engage one or more couplers 235a-235c disposed on distal face 223 upon seating the motor cartridge 500 within cavity 224. Universal couplers 540a-540c are actuatable upon actuation of one or more actuation buttons 245a-245c. Each actuation button, e.g., button 245a, couples to a corresponding universal coupler, e.g., coupler 540a, via electrical or electromechanical connection through the engagement of corresponding electrical or electromechanical interfaces, e.g., interface 225a and 545a. Stapler 200 may have any number of couplers 235a-235c to perform any number of functions, e.g., open and close the jaw members 282, 284, rotate the jaw members 282, 284, articulate the shaft 212, fire a staple (not shown) into tissue, actuate a knife, e.g., knife 156, between jaw members 282, 284, and energize the jaw members 282, 284 to treat tissue.

The universal nature of the motor cartridge 500 with the plurality of couplers 540a-540c enables instrument manufacturers the ability to utilize the plurality of couplers 540a-540c to perform any number of tasks for various surgical instrumentation. Moreover, instruments may be manufactured such that two or more couplers, e.g., 540a, 540b, may be actuated at the same time upon actuation of one or more actuation buttons 245a-245c, enabling more versatility with the universal motor cartridge 500.

Electrical cable 530 extends from the motor cartridge 500 and is adapted to connect to an electrical energy source, e.g., generator 750. Cable 530 may be selectively engageable with motor cartridge 500 at connection 535. With particular reference to stapler 200, cable 530 may be disconnected at connection 535 since stapler 200 includes an internal power supply 275 or supplemental power supply (shown in phantom). Power supply 275 powers the motor cartridge 500 for activation of the various couplers 545a-545c of motor cartridge 500 and couplers 235a-235c of stapler 200 to allow a the surgeon to rotate/articulate the shaft 212, open/close the jaw members 282, 284, fire staples (not shown) to staple tissue, cut tissue disposed between the jaw members 282, 284 and, in some instances, provide electrical energy to the jaw members 282, 284 if the stapler 200 is configured as such. In addition to having an internal motor supply, the stapler 200 may be configured to connect to an external electrical source, e.g., generator 750, depending upon a particular purpose.

The various switches 245a-245c may be configured as per user preference upon startup. For example, switch 245a may be a toggle switch and may be configured to open and close the jaw members 282, 284 upon actuation thereof. Switch 245b may be used to rotate the shaft 212 in the direction "R" or articulate in direction "A" and switch 245c may be utilized to fire the stapler 200 to deform staples (not shown) into tissue and/or advance a knife disposed on the firing mechanism at the same time. Alternatively, another switch (not shown) may be disposed on the housing 220 or handle 250 and used to advance the knife independent of the firing mechanism to deform the staples. Motor cartridge 500 may also include one or more switches 542 which may be customized for any purpose (rotation, articulation, opening/closing jaw members 282, 284, a safety switch, energizing jaw members 282, 284, etc.).

In use, the user unpacks a sterilized disposable stapler 200 and couples the reusable universal motor cartridge 500 to the stapler 200 as described above. Once coupled, the user may be prompted to configure the stapler 200 for use or use the default settings depending upon a particular purpose. A user's profile may be stored in the motor cartridge 500 for a given instrument. After use, the disposable portion, e.g., stapler 200, is discarded or stored for sterilization and refurbishment. The reusable motor cartridge 500, once sterilized, may be used again with another forceps 10, vessel sealer 400 (FIG. 3) or new stapler 200 (FIG. 2).

Turning now to FIG. 3, the universal motor cartridge 500 may be used with a disposable vessel sealer 400 in much the same fashion as described above. Vessel sealer 400 includes a housing 420 having a shaft 412 extending therefrom which has a distal end 416 dimensioned to mechanically engage the end effector assembly 401 and a proximal end 414 which mechanically engages the housing 420. The proximal end 414 of shaft 412 is received within the housing 420. A handle 450 depends from housing 420 and is configured to support a variety of actuation buttons, levers or switches 445a-445c on handle 450, the purposes of which are explain in detail below. Handle 450 is integrally associated with housing 420. End effector assembly 401 includes a first jaw member 410 movable relative to a second jaw member 420 upon actuation of one of the actuation buttons, e.g., actuation button 445a. A cavity 424 is defined between an upper surface 422 of the housing 420 and a distal face 423 extending therefrom. Cavity 424 is configured to receive the motor cartridge 500 therein. One or more mechanical interfaces or alignment features 427a, 427b are utilized to operably engage and align 200 with corresponding interfaces 529a, 529b disposed on the motor cartridge 500 to align and secure the motor cartridge 500 within cavity 424. Mechanical interfaces 427a, 427b may include inter-cooperating components, slide rails, tongue and groove arrangements, magnets, etc.

Upper surface 422 supports a plurality of electrical or electromechanical interfaces 425a-425c thereon which are configured to align and operably engage a plurality of electrical or electromechanical interfaces 545a-545c (shown in phantom) disposed on the motor cartridge 500. Mechanical interfaces 427a, 427b and 529a, 529b help to align the electrical or electromechanical interfaces 425a-425c and 545a-545c. It is important to note that the number of electrical or electromechanical interfaces 425a-425c associated with upper surface 422 of housing 420 may be different than the number of electrical or electromechanical interfaces 545a-545c associated with motor cartridge 500. In other words, not all instruments that are adapted to couple to the universal motor cartridge 500 will be adapted to utilize all of the electrical or electromechanical interfaces 545a-545c for specific purposes.

Motor cartridge 500 also includes the plurality of universal couplers 540a-540c disposed on the distal surface 522 thereof that are configured to operably engage one or more couplers 435a-435c disposed on distal face 423 upon seating the motor cartridge 500 within cavity 424. Universal couplers 540a-540c are actuatable upon actuation of one or more actuation buttons 445a-445c. Each actuation button, e.g., button 445a, couples to a corresponding universal coupler, e.g., coupler 540a, via electrical or electromechanical connection through the engagement of corresponding electrical or electromechanical interfaces, e.g., interface 425a and 545a. Vessel sealer 400 may have any number of couplers 435a-435c to perform any number of functions, e.g., open and close the jaw members 410, 420, actuate a knife, e.g., knife 156, between jaw members 410, 420, and energize the jaw members 410, 420 to treat tissue.

The various switches 445a-445c may be configured as per user preference upon startup. For example, switch 445a may be a toggle switch and may be configured to open and close the jaw members 410, 420 upon actuation thereof. Switch 445b may be used to energize the jaw members 410, 420 to treat tissue, and switch 445c may be utilized to advance the knife, e.g., knife 156, to cut tissue disposed between jaw members 410, 420. Alternatively, another switch may be disposed on the housing 420 or handle 450 and perform an additional function. Motor cartridge 500 may also include one or more switches 542 which may be customized for any purpose including redundancy (opening/closing jaw members 110, 120, a safety switch, knife actuation, energizing jaw members 410, 420, etc.).

In use, the user unpacks a sterilized disposable vessel sealer 400 and couples the reusable universal motor cartridge 500 to the vessel sealer 400 as described above. Once coupled, the user may be prompted to configure the vessel sealer 400 for use or use the default settings depending upon a particular purpose. A user's profile may be stored in the motor cartridge 500 for a given instrument. After use, the disposable portion, e.g., vessel sealer 400, is discarded or stored for sterilization and refurbishment. The reusable motor cartridge 500, once sterilized, may be used again with a forceps 10, a new vessel sealer 400 (FIG. 3) or stapler 200 (FIG. 2).

Although described above in terms of generally electrical energy being transferred from the reusable motor cartridge 500 to the disposable portion, e.g., vessel sealer 400, it is contemplated to enclose the entire motor cartridge 500 into the reusable section with only mechanical interfaces, e.g., gears, drive shafts, pins, etc., interfacing with the disposable portion, e.g., vessel sealer 400. It is envisioned that configuring the reusable motor cartridge 500 and disposable portion in this fashion would significantly reduce the cost of the disposable portion.

It is contemplated that configuring the reusable motor cartridge 500 with an electrical motor and various electrical actuators has many additional benefits as far as instrument precision and accuracy with respect to the disposable components, e.g., the ability to precisely control and measure aspects relating to jaw pressure, jaw tension, jaw gap, blade force etc., simply based on electrical feedback. By monitoring the current required by the motor for completing a task (or at set point of a task) and comparing the same to a table or algorithm, parameters of the tissue or instrument may be precisely determined, e.g., jaw gap, tissue reaction, jaw tension, blade parameters, etc., during all aspects of the sealing process.

For example, forces countering the motor (increasing current draw) would enable the algorithm to determine if a user is pulling on tissue, which may adversely affect the seal quality. During initial grasping (a potential set point), by measuring current draw the algorithm may be able to measure if the jaw members are overstuffed with tissue, e.g., a sharp increase in current at a particular set point. In this instance, the motor may be configured to adapt to a "slow close" algorithm to improve tissue desiccation and sealing of large tissue bundles.

In other embodiments, by measuring the motor force and comparing jaw gap, different tissue types may be identified simply using current. If there is a spike in current to open the jaw members, the tissue may be sticking. By measuring current at another set point, e.g., the current for driving the blade through tissue, feedback may be generated relating to the level of tissue desiccation or possible eschar in the blade slot. Moreover, continuing to monitor the current during blade retraction can provide feedback regarding the blade being stuck or simply that the blade has fully returned to a safety position to allow opening of the jaw members.

As a result of configuring the reusable motor cartridge 500 with electrical motors and actuators, more intelligent feedback algorithms combined with RF energy algorithms may be designed to improve stapling or vessel sealing performance. In addition, it is contemplated that the universal motor cartridge may be configured to deploy one or more secondary electrical devices such as a hook, e.g., monopolar or bipolar hook, a suction or irrigation tube or any other electrical or non-electrical implement.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as examples of particular embodiments. Those skilled in the art will envision other modifications within the scope and spirit of the claims appended hereto.

Although the foregoing disclosure has been described in some detail by way of illustration and example, for purposes of clarity or understanding, it will be obvious that certain changes and modifications may be practiced within the scope of the appended claims.

The invention may be described by reference to the following numbered paragraphs:-
1. A universal motor cartridge for a surgical instrument, comprising:
   a reusable housing having a distal face at a distal end thereof and a bottom surface, the housing configured to securely engage a disposable surgical instrument;
   a plurality of couplers disposed on the distal face, at least one of the plurality of couplers adapted to engage a corresponding coupler disposed on the surgical instrument upon engagement of the housing thereto;
   a plurality of electrical interfaces disposed on the bottom surface, at least one of the plurality of electrical interfaces adapted to electrically engage a corresponding electrical interface disposed on a mating surface of the surgical instrument, such that, upon activation of at least one switch disposed on the surgical instrument, the corresponding electrical interface on the surgical instrument communicates with the at least one of the plurality of electrical interfaces on the bottom surface of the housing, which, in turn, actuates the at least one of the plurality of couplers to engage and actuate at least one corresponding coupler on the surgical instrument, wherein the at least one corresponding coupler on the surgical instrument, upon actuation thereof, causes the surgical instrument to at least one of: rotate or articulate a shaft associated therewith, open or close a pair of jaw members associated therewith, deform a plurality of staples associated therewith, move a knife associated therewith to cut tissue, or energize an end effector associated therewith.
2. The universal motor cartridge for a surgical instrument according to paragraph 1, wherein the housing is configured to securely engage an electrosurgical forceps having a plurality of switches associated therewith, each switch of the plurality of switches, upon activation thereof, communicating with a corresponding one of the plurality of electrical interfaces on the electrosurgical forceps which, in turn, communicates with a corresponding electrical interface on the bottom surface of the housing to actuate a corresponding coupler on the housing which, in turn, actuates a corresponding coupler on the electrosurgical forceps to cause the electrosurgical forceps to at least one of: rotate or articulate a shaft associated therewith, open or close a pair of jaw members associated therewith, move a knife associated therewith to cut tissue, or energize an end effector associated therewith.
3. The universal motor cartridge for a surgical instrument according to paragraph 1, wherein the housing is configured to securely engage a stapling device having a plurality of switches associated therewith, each switch of the plurality of switches, upon activation thereof, communicating with a corresponding one of the plurality of electrical interfaces on the stapling device which, in turn, communicates with a corresponding electrical interface on the bottom surface of the housing to actuate a corresponding coupler on the housing which, in turn, actuates a corresponding coupler on the stapling device to cause the stapling device to at least one of: rotate or articulate a shaft associated therewith, open or close a pair of jaw members associated therewith, move a knife associated therewith to cut tissue, or deform a plurality of staples associated therewith.
4. The universal motor cartridge for a surgical instrument according to paragraph 1, wherein the motor cartridge is adapted to connect to an electrical energy source.
5. The universal motor cartridge for a surgical instrument according to paragraph 1, wherein the motor cartridge is adapted to connect to an internal electrical energy source.
6. The universal motor cartridge for a surgical instrument according to paragraph 1, wherein the housing includes at least one mechanical interface configured to align and securely engage a corresponding mechanical interface disposed on the surgical instrument.
7. The universal motor cartridge for a surgical instrument according to paragraph 1, wherein the housing includes at least one switch disposed thereon that actuates a corresponding coupler on the housing which, in turn, actuates a corresponding coupler on the surgical instrument to cause the surgical instrument to at least one of: rotate or articulate a shaft associated therewith, open or close a pair of jaw members associated therewith, deform a plurality of staples associated therewith, move a knife associated therewith to cut tissue, or energize an end effector associated therewith.
8. The universal motor cartridge for a surgical instrument according to paragraph 1, wherein the motor assembly is configured to identify the type of surgical instrument upon engagement therewith and configure at least one of the plurality of couplers disposed on the distal face in accordance thereto.
9. The universal motor cartridge for a surgical instrument according to paragraph 1, wherein the surgical instrument includes two or more switches disposed thereon, activation of a first of the two switches actuates a first coupler on the housing which, in turn, actuates a first coupler on the surgical instrument to cause the surgical instrument to perform a first function and activation of a second of the two or more switches actuates a second coupler on the housing which, in turn, actuates a second coupler on the surgical instrument to cause the surgical instrument to perform a second function and activation of a both the first and second switches of the two or more switches actuates a third coupler on the housing which, in turn, actuates a third coupler on the surgical instrument to cause the surgical instrument to perform a third function.
10. The universal motor cartridge for a surgical instrument according to paragraph 9, wherein the first, second and third functions of the surgical instrument include: rotation or articulation of a shaft associated with the surgical instrument, opening or closing a pair of jaw members associated with the surgical instrument, deforming a plurality of staples associated with the surgical instrument, moving a knife associated with the surgical instrument to cut tissue, or energizing an end effector associated with the surgical instrument.
11. The universal motor cartridge for a surgical instrument according to paragraph 9, wherein simultaneous actuation of the first and second switches on the surgical instrument actuates the third coupler on the housing which, in turn, actuates the third coupler on the surgical instrument to cause the surgical instrument to perform the third function.

## Claims

1. A universal motor cartridge for a surgical instrument, comprising:
a reusable housing having a distal face at a distal end thereof and a bottom surface, the housing configured to securely engage a disposable surgical instrument;
a plurality of couplers disposed on the distal face, at least one of the plurality of couplers adapted to engage a corresponding coupler disposed on the surgical instrument upon engagement of the housing thereto;
a plurality of electrical interfaces disposed on the bottom surface, at least one of the plurality of electrical interfaces adapted to electrically engage a corresponding electrical interface disposed on a mating surface of the surgical instrument, such that, upon activation of at least one switch disposed on the surgical instrument, the corresponding electrical interface on the surgical instrument communicates with the at least one of the plurality of electrical interfaces on the bottom surface of the housing, which, in turn, actuates the at least one of the plurality of couplers to engage and actuate at least one corresponding coupler on the surgical instrument, wherein the at least one corresponding coupler on the surgical instrument, upon actuation thereof, causes the surgical instrument to at least one of: rotate or articulate a shaft associated therewith, open or close a pair of jaw members associated therewith, deform a plurality of staples associated therewith, move a knife associated therewith to cut tissue, or energize an end effector associated therewith.

2. The universal motor cartridge for a surgical instrument according to claim 1, wherein the housing is configured to securely engage an electrosurgical forceps having a plurality of switches associated therewith, each switch of the plurality of switches, upon activation thereof, communicating with a corresponding one of the plurality of electrical interfaces on the electrosurgical forceps which, in turn, communicates with a corresponding electrical interface on the bottom surface of the housing to actuate a corresponding coupler on the housing which, in turn, actuates a corresponding coupler on the electrosurgical forceps to cause the electrosurgical forceps to at least one of: rotate or articulate a shaft associated therewith, open or close a pair of jaw members associated therewith, move a knife associated therewith to cut tissue, or energize an end effector associated therewith.

3. The universal motor cartridge for a surgical instrument according to claim 1, wherein the housing is configured to securely engage a stapling device having a plurality of switches associated therewith, each switch of the plurality of switches, upon activation thereof, communicating with a corresponding one of the plurality of electrical interfaces on the stapling device which, in turn, communicates with a corresponding electrical interface on the bottom surface of the housing to actuate a corresponding coupler on the housing which, in turn, actuates a corresponding coupler on the stapling device to cause the stapling device to at least one of: rotate or articulate a shaft associated therewith, open or close a pair of jaw members associated therewith, move a knife associated therewith to cut tissue, or deform a plurality of staples associated therewith.

4. The universal motor cartridge for a surgical instrument according to claim 1, 2 or 3 wherein the motor cartridge is adapted to connect to an electrical energy source.

5. The universal motor cartridge for a surgical instrument according to claim 1, 2 or 3 wherein the motor cartridge is adapted to connect to an internal electrical energy source.

6. The universal motor cartridge for a surgical instrument according to any preceding claim, wherein the housing includes at least one mechanical interface configured to align and securely engage a corresponding mechanical interface disposed on the surgical instrument.

7. The universal motor cartridge for a surgical instrument according to any preceding claim, wherein the housing includes at least one switch disposed thereon that actuates a corresponding coupler on the housing which, in turn, actuates a corresponding coupler on the surgical instrument to cause the surgical instrument to at least one of: rotate or articulate a shaft associated therewith, open or close a pair of jaw members associated therewith, deform a plurality of staples associated therewith, move a knife associated therewith to cut tissue, or energize an end effector associated therewith.

8. The universal motor cartridge for a surgical instrument according to any preceding claim, wherein the motor assembly is configured to identify the type of surgical instrument upon engagement therewith and configure at least one of the plurality of couplers disposed on the distal face in accordance thereto.

9. The universal motor cartridge for a surgical instrument according to any preceding claim, wherein the surgical instrument includes two or more switches disposed thereon, activation of a first of the two switches actuates a first coupler on the housing which, in turn, actuates a first coupler on the surgical instrument to cause the surgical instrument to perform a first function and activation of a second of the two or more switches actuates a second coupler on the housing which, in turn, actuates a second coupler on the surgical instrument to cause the surgical instrument to perform a second function and activation of a both the first and second switches of the two or more switches actuates a third coupler on the housing which, in turn, actuates a third coupler on the surgical instrument to cause the surgical instrument to perform a third function.

10. The universal motor cartridge for a surgical instrument according to claim 9, wherein the first, second and third functions of the surgical instrument include: rotation or articulation of a shaft associated with the surgical instrument, opening or closing a pair of jaw members associated with the surgical instrument, deforming a plurality of staples associated with the surgical instrument, moving a knife associated with the surgical instrument to cut tissue, or energizing an end effector associated with the surgical instrument.

11. The universal motor cartridge for a surgical instrument according to claim 9 or 10, wherein simultaneous actuation of the first and second switches on the surgical instrument actuates the third coupler on the housing which, in turn, actuates the third coupler on the surgical instrument to cause the surgical instrument to perform the third function.
